Europäisches Patentamt

European Patent Office

Office européen des brevets

(11) Publication number: **0 505 579 A1**

## EUROPEAN PATENT APPLICATION
### published in accordance with Art. 158(3) EPC

(21) Application number: 91919280.7

(22) Date of filing: 09.10.91

(86) International application number: PCT/JP91/01382

(87) International publication number: WO 92/06722 (30.04.92 92/10)

(51) Int. Cl.5: **A61M 5/178**, A61M 5/24, A61M 5/31, A61M 5/315

(30) Priority: **15.10.90 JP 275704/90**

(43) Date of publication of application: **30.09.92 Bulletin 92/40**

(84) Designated Contracting States: **AT BE CH DE DK ES FR GB GR IT LI LU NL SE**

(71) Applicant: SEIKAGAKU KOGYO CO., LTD. 1-5, Nihonbashi-Honcho 2-chome Chuo-ku Tokyo 103(JP)

(72) Inventor: **KANEKO, Kazunobu** **15-16, Okusawa 7-chome** **Setagaya-ku, Tokyo 158(JP)**

(74) Representative: **Füchsle, Klaus, Dipl.-Ing. et al** **Hoffmann . Eitle & Partner Patentanwälte** **Arabellastrasse 4** **W-8000 München 81(DE)**

(54) **INJECTOR SUPPLIED WITH MEDICINE.**

(57) An injector supplied with a feed quantity of a medicine (5) sealed inside the cylinder (1) thereof before used for injection and, in particular, to an injector supplied with a medicine requiring addition of liquid thereto at the time of injection. A characteristic of the invention is such that a plunger (4) is formed longer than a distance of movement thereof required for sucking the liquid into the cylinder (1). This structure can prevent the medicine and the liquid from being polluted by bacteria because the inner wall of the cylinder (1) is shielded by the plunger (4) to be kept in a sterilized state before brought into contact with the medicine and the liquid being sucked.

Fig. 1

[Technical Field]

This invention relates to an injector supplied with medicine having a predetermined amount of medicine beforehand sealed in a cylinder tube, and more particularly to an injector supplied with medicine which is used either in the case where the sealed medicine is a liquid, and it is necessary to further add a liquid of a different medicine when applying an injection, or in the case where the sealed medicine is powder, such for example as a frozen dried form of liquid medicine, and it is returned to the liquid medicine by the addition of a solvent or a liquid of a medicine when applying an injection.

[Background Art]

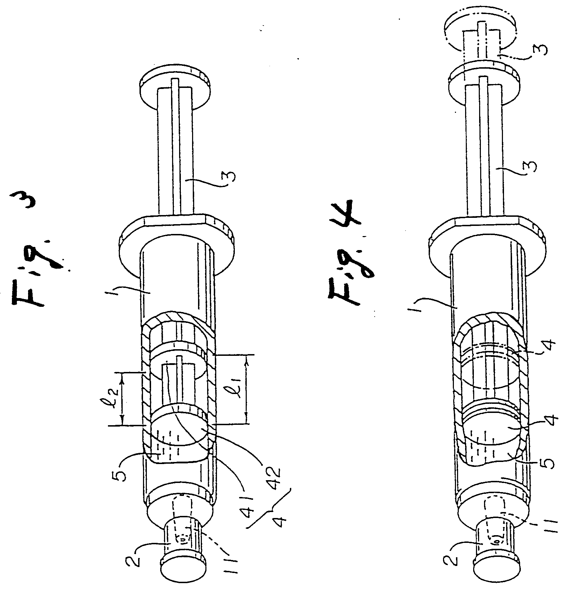

Recently, from a sanitary point of view, disposable injectors have been widely used, and particularly an injector supplied with medicine having a predetermined amount of medicine sealed therein is quite convenient and sanitary because a medicine contained in an ampul or the like does not need to be drawn into the injector when applying an injection. One known example of injector supplied with medicine is shown in Fig. 4. In this injector supplied with medicine, a cap 2 is press-fitted on an injector needle mounting portion 11 projectingly formed at a distal end of a cylinder tube 1, and a plunger 4 fixedly secured to a distal end of a plunger rod 3 is fitted to a predetermined position from the proximal end side of the cylinder tube 1, and a predetermined amount of a liquid 5 composed of a liquid medicine is sealed. An injection can be applied by a simple operation in which the cap 2 is removed to expose the injector needle mounting portion 11, and then an injector needle is connected thereto.

Also, such an injector supplied with medicine has an advantage that by beforehand sterilizing the cylinder tube 1, the cap 2 and the plunger 4, the medicine sealed in the cylinder tube can be kept in a sterilized condition throughout the distribution and storage process without putting the injector in a sterilization bag or the like.

However, in the above conventional injector supplied with medicine, that section of its proximal portion extending from the plunger 4 toward its proximal end is kept exposed, and there is a risk that this section may be contaminated by bacilli or the like during the distribution and storage process. Therefore, when a predetermined amount of liquid is required to be further added to the sealed medicine 5 when applying an injection, the plunger must be moved to the contaminated portion of the cylinder tube so as to suck the predetermined amount of liquid, thus posing a problem that the medicine 5 and the liquid are brought into contact with the contaminated portion of the inner wall of the cylinder tube 1. In order to avoid such a situation, although it can be considered to adopt means by which the whole of the injector having the medicine sealed therein is again sterilized, and then is accommodated within a sterilization bag, such operation is not easy, and particularly the medicine is, in general, changed in nature or decomposed by heat, light rays, electromagnetic waves and the like, and therefore the sterilization after the sealing is difficult. Further, a conventionally-used method depending on a sterilization gas is not desirable from the viewpoint of safety.

The present invention has been made in view of the above circumstances, and an object of the invention is to provide an injector supplied with medicine which, of course, prevents the contamination by bacilli or the like which would occur as a result of sucking a liquid when applying an injection, and also does not require a cumbersome sterilization treatment at a final stage of the production process.

[Disclosure of the Invention]

In order to solve the above problems with an injector supplied with medicine wherein a cap is attached to an injector needle mounting portion projectingly formed at a distal end of a cylinder tube; a plunger on a distal end of a plunger rod is fitted to a predetermined position from a proximal end side, and a predetermined amount of medicine is sealed; when applying an injection, the cap is removed from the injector needle mounting portion, and an injector needle is connected to this exposed injector needle mounting portion, and the plunger is moved a predetermined distance toward the proximal end side to draw a predetermined amount of liquid into the cylinder tube, there is provided a first means in which the length of the plunger is at least longer than the distance of movement for drawing the liquid into the cylinder tube. According to a second means, the plunger comprises a plunger member provided at the distal end of the plunger rod, and another plunger member provided at a proximal end thereof and spaced a predetermined distance from the plunger member, and the distance between the plunger members is at least longer than the distance of movement of the plunger for drawing the liquid into the cylinder tube.

The inner wall portion of the cylinder tube which is brought into contact with the medicine and the liquid upon movement of the plunger for drawing the liquid is beforehand shut off by the plunger and is kept in a sterilized condition, and therefore the contamination caused by the drawing of the liquid is prevented.

[Brief Description of the Drawings]

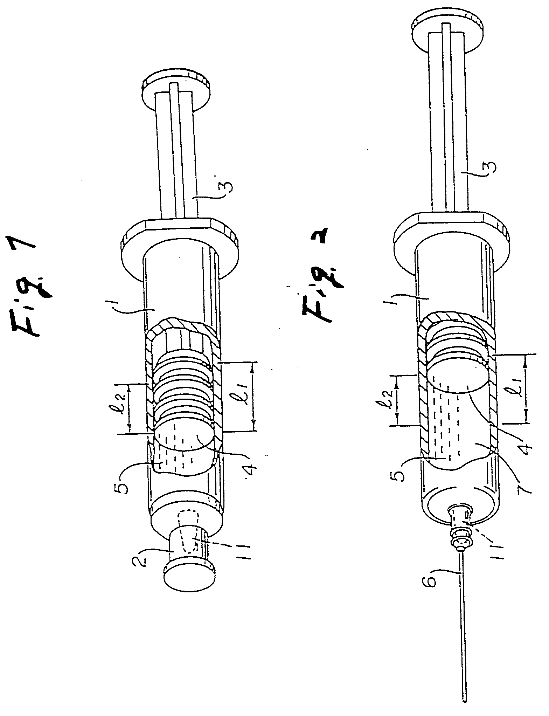

Figs. 1 and 2 show one embodiment of a first invention; Fig. 1 is a partly-broken, perspective view; Fig. 2 is a partly-broken, perspective view showing a condition of use; Fig. 3 is a partly-broken, perspective view of one embodiment of a second invention; and Fig. 4 is a partly-broken, perspective view of the conventional example.

1 ... cylinder tube, 2 ... cap, 3 ... plunger rod, 4 ... plunger, 5 ... medicine, 6 ... injector needle, 11 ... injector needle mounting portion, 41, 42 .. plunger member.

[Best Mode for carrying out the Invention]

Embodiments of the present invention will now be described with reference to the drawings.

Those portions similar in construction to those of the above-mentioned conventional example are designated by identical reference numerals, respectively, when explaining these.

Figs. 1 and 2 show one embodiment of a first invention. A cap 2 made, for example, of butyl rubber is press-fitted on an injector needle mounting portion 11 projectingly formed at a distal end of a graduated cylinder tube 1 of glass. A plunger 4, fixedly secured to a distal end of a plunger rod 3 inserted from the proximal end of the cylinder tube 1, is fitted to a predetermined position, and a predetermined amount of liquid medicine 5 is sealed therein. The length $\ell_1$ of the plunger 4 is at least longer than the distance $\ell_2$ of movement of the plunger 4 for drawing a liquid into the cylinder tube 1 when applying an injection.

For manufacturing this embodiment, a sterilization treatment, such as a heat treatment, is first applied to each of the constituent parts, and then the plunger 4 fitted in the cylinder tube 1 is moved to a predetermined position to fill a predetermined amount of medicine 5 therein, and the cap 2 is attached to the injector needle mounting portion 11. In this condition, it is distributed and stored without using a bag sterilization. Then, when it is to be used, the cap 2 is removed, and an injector needle 6 is connected to this exposed injector needle mounting portion 11 as shown in Fig. 2, and the plunger rod 3 is grasped, and the plunger 4 is moved to a predetermined position toward the proximal end, so that a predetermined amount of liquid is drawn into the cylinder tube 1 in accordance with the graduation on the cylinder tube 1, and in this condition an injection is applied to a human body or the like. At this time, in this embodiment, the length $\ell_1$ of the plunger 4 is longer than the distance $\ell_2$ of movement of the plunger 4 for drawing the liquid into the cylinder tube 1. Therefore, the inner wall portion of the cylinder tube 1 serving as a new medicine-receiving portion is beforehand shut off by the plunger 4, and therefore is kept in a sterilized condition until when applying the injection, and the medicine 5 and the liquid will not be contaminated when sucking the liquid.

Fig. 3 shows one embodiment of a second invention, and the overall construction thereof is generally similar to that of the above embodiment of the first invention. However, a plunger 4 comprises a plunger member 41 provided at a distal end of a plunger rod 3 so as to seal a liquid medicine 5, and another plunger member 42 provided at the proximal end of the plunger. The distance $\ell_1$ between the plunger members 41, 42 is at least longer than the distance $\ell_2$ of movement of the plunger 4 for drawing a liquid into a cylinder tube 1 when applying an injection.

The manufacture, the manner of use and the operation of this embodiment are similar to those of the above embodiment of the first invention, but unlike the above embodiment of the first invention, the plunger 4 is constituted by the two thin plunger members 41, 42, and therefore the area of contact with the cylinder tube 1 is small, which provides an advantage that the movement of the plunger 4 can be smoothly effected with a less frictional resistance, thereby achieving a good operability.

In either of the above embodiments, the predetermined amount of medicine 5 sealed in the cylinder tube 1 is in the form of a liquid, and the predetermined amount of liquid composed of a medicine is sucked when applying the injection. However, the invention can also be performed in the case of many other kinds of liquids, as described in the above embodiments, for example, in the case where the predetermined amount of medicine 5 sealed in the cylinder tube 1 is powdered or granular, and a predetermined amount of a solvent or a liquid is sucked when applying an injection.

[Capability of Exploitation in Industry]

In the present invention having the above construction, taking it into consideration that the liquid is added to the sealed medicine when applying an injection, the interior of the cylinder tube is beforehand kept in a sterilized condition by the plunger over a wide range, and therefore when sucking the liquid, the medicine and the liquid will not be contaminated by bacilli or the like deposited on the inner surface of the cylinder tube or the plunger rod. Therefore, it can, of course, be used in a medical field and the field of animal tests, and can also be applied to a reagent for an ordinary chemical test or for clinical examination which reagent need adjustment when used, and must be prevented from contamination by a mixture. After the medicine is sealed, the injector does not need to be put into a bag after applying a sterilization treatment thereto, but it can be distributed and stored in this condition, and therefore is quite convenient and economical. Further, the length of the plunger is not limited to the distance of movement for drawing the liquid into the cylinder tube, but can be made longer than the length which the mixture of the medicine and the liquid occupies within the cylinder tube. In this case, when the mixture is all pushed out by moving the plunger to the distal end, the proximal end of the plunger is disposed at a position spaced toward the proximal end side from the proximal end of the receiving portion of the cylinder tube in which the mixture has been filled, thereby shutting off the portion with which the mixture has been contacted. Therefore, if either the medicine or the liquid is a contaminating substance, such as a radioactive substance and a cancer-inducing substance, there is no fear that the contamination will spread from the used injector to the exterior, and therefore the degree of safety is high.

## Claims

1.  An injector supplied with medicine wherein a cap is attached to an injector needle mounting portion projectingly formed at a distal end of a cylinder tube; a plunger on a distal end of a plunger rod is fitted to a predetermined position from a proximal end side, and a predetermined amount of medicine is sealed; when applying an injection, said cap is removed from said injector needle mounting portion, and an injector needle is connected to this exposed injector needle mounting portion, and said plunger is moved a predetermined distance toward the proximal end side to draw a predetermined amount of liquid into said cylinder tube; CHARACTERIZED in that the length of said plunger is at least longer than the distance of movement for drawing said liquid into said cylinder tube.

2.  An injector supplied with medicine wherein a cap is attached to an injector needle mounting portion projectingly formed at a distal end of a cylinder tube; a plunger on a distal end of a plunger rod is fitted to a predetermined position from a proximal end side, and a predetermined amount of medicine is sealed; when applying an injection, said cap is removed from said injector needle mounting portion, and an injector needle is connected to this exposed injector needle mounting portion, and said plunger is moved a predetermined distance toward the proximal end side to draw a predetermined amount of liquid into said cylinder tube; CHARACTERIZED in that said plunger comprises a plunger member provided at the distal end of the plunger rod, and another plunger member provided at a proximal end thereof and spaced a predetermined distance from said plunger member; and the distance between said plunger members is at least longer than the distance of movement of said plunger for drawing said liquid into said cylinder tube.

EP 0 505 579 A1

# Fig. 1

# Fig. 2

Fig. 3

Fig. 4

EP 0 505 579 A1

# INTERNATIONAL SEARCH REPORT

International Application No PCT/JP91/01382

**I. CLASSIFICATION OF SUBJECT MATTER** (if several classification symbols apply, indicate all) *

According to International Patent Classification (IPC) or to both National Classification and IPC

Int. Cl$^5$  A61M5/178, A61M5/24, A61M5/31, 315

**II. FIELDS SEARCHED**

| Minimum Documentation Searched [7] | |
|---|---|
| Classification System | Classification Symbols |
| | |

Documentation Searched other than Minimum Documentation
to the Extent that such Documents are Included in the Fields Searched [8]

Jitsuyo Shinan Koho                    1926 - 1990
Kokai Jitsuyo Shinan Koho              1971 - 1990

**III. DOCUMENTS CONSIDERED TO BE RELEVANT [9]**

| Category * | Citation of Document, [11] with indication, where appropriate, of the relevant passages [12] | Relevant to Claim No. [13] |
|---|---|---|
| Y | JP, B2, 60-13705 (Becton Dickinson and Co.), April 9, 1985 (09. 04. 85) Full descriptions, all drawings & US, A, 4074715 & FR, B1, 2368970 & GB, A, 1590095 & DE, C2, 2748776 | 1, 2 |
| Y | JP, U, 57-176244 (Takeshi Eguchi), November 8, 1982 (08. 11. 82), Full descriptions, all drawings (Family: none) | 1, 2 |

\* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| November 22, 1991 (22. 11. 91) | December 9, 1991 (09. 12. 91) |

| International Searching Authority | Signature of Authorized Officer |
|---|---|
| Japanese Patent Office | |

Form PCT/ISA/210 (second sheet) (January 1985)